Europäisches Patentamt

European Patent Office

Office européen des brevets

(19)

(11) Publication number: **0 044 723**
A2

(12) **EUROPEAN PATENT APPLICATION**

(21) Application number: **81303287.7**

(22) Date of filing: **16.07.81**

(51) Int. Cl.³: **A 01 H 1/02**

(30) Priority: **17.07.80 US 169876**
**17.07.80 US 169875**
**17.07.80 US 169874**

(43) Date of publication of application:
**27.01.82 Bulletin 82/4**

(84) Designated Contracting States:
**DE FR GB IT NL**

(71) Applicant: **AGRIGENETICS CORPORATION**
**1726 Cole Boulevard**
**Denver Colorado 80401(US)**

(72) Inventor: **Lawrence, Robert Howard, Jr.**
**708 West South Street**
**Kalamazoo Michigan 49007(US)**

(72) Inventor: **Hill, Phillip Eugene**
**781 Welburn Avenue**
**Gilroy California 95020(US)**

(74) Representative: **Senior, Janet et al,**
**Abel & Imray Northumberland House 303-306 High**
**Holborn**
**London WC1V 7LH(GB)**

(54) A process for the rapid development of hybrid plants and commercial production of hybrid seed.

(57) Hybrid plants and seeds are produced in increased yields. The process combines the techniques of sexual reproduction to develop new hybrids and seeds, and cloning of original parent plants to provide large scale replication of the original crosses. New hybrid seed varieties can be developed and readied for market in as little as three years, compared to the conventional eight to twelve years normally required for commercial production. Plant breeders are no longer limited to using homozygous parents in the production of new plant hybrids. The process provides a special advantage in the production of increased yields of high purity Brassica seeds. In this instance cloning parents that are maximally self-incompatible results in a parental line consisting of cloned plants that are essentially sib-incompatible; thus, the process eliminates the difficulties of conventional methods which require bud-pollination to maintain sib-incompatible parent lines through inbreeding.

EP 0 044 723 A2

## A PROCESS FOR THE RAPID DEVELOPMENT OF HYBRIDS PLANTS AND COMMERCIAL PRODUCTION OF HYBRID SEED

## TABLE OF CONTENTS

1.   Cross References to Related Applications
2.   Background of the Invention
     2.1  Field of the Invention
     2.2  Background of the Invention
3.   Summary of the Invention
4.   Description of the Invention
     4.1  Development of Breeding Strategy
     4.2  Selection of Individual Parent Plant
     4.3  Crossing of Individual Original Parent Plants
     4.4  Obtaining Clonable Explant Tissue
     4.5  Optional Storage of Explants or Shoots
     4.6  Cloning Explant Tissue
     4.7  Selecting Optimum Hybrids
     4.8  Crossing Cloned Parental Lines
     4.9  Verification by Field Trials
     4.10 Commercial Production
     4.11 Applications
     4.12 Example 1 − Tomatoes
          4.121  Selection of Individual Parent Plants
          4.122  Crossing of Individual Parent Plants
          4.123  Obtaining Clonable Explant Tissue
          4.124  Optional Storage of Explants or Shoots
          4.125  Cloning Explant Tissue
          4.126  Planting of Plantlets
          4.127  Crossing Original Parent Plants
          4.128  Crossing Cloned Parental Lines
          4.129  Verification by Field Trial
          4.1291 Commercial Production

## TABLE OF CONTENTS

4.13 Example 2 − Cabbage

    4.131  Schedule for Commercial Production of High
            Purity Hybrid Seed

    4.132  Clonal Propagation of Cabbage

4.14 Example 3 − Cauliflower

    4.141  Schedule for Commercial Production of
           Hybrid Seed

    4.142  Clonal Propagation of Cauliflower

## A PROCESS FOR THE RAPID DEVELOPMENT OF HYBRIDS PLANTS AND COMMERCIAL PRODUCTION OF HYBRID SEEDS

### 1. CROSS REFERENCES TO OTHER APPLICATIONS

This invention is disclosed in copending United States patent applications of the present inventors:

Ser. No. 169,876, filed July 17, 1980; Ser. No. 169,875, filed July 17, 1980; and Ser. No. 169,874, filed July 17, 1980.

### 2. BACKGROUND OF THE INVENTION

#### 2.1 Field of the Invention

This invention relates to the rapid development and commercial production of hybrid seeds and plants therefrom including melons, vegetables, grains, forages, fibre crops, and other monoecious species. New hybrid plants with improved properties are developed rapidly and hybrid seeds thereof are produced in increased yields. The process, which combines the techniques of sexual reproduction and cloning (or vegetative propagation), imposes no limitations on the homozygosity or heterozygosity of the parents. An additional advantage is obtained when the process is applied to the production of high purity Brassica seeds in increased yields; the process eliminates the conventional necessity of labor-intensive bud-pollination to produce sib-incompatible parent plant lines.

## 2.2  Background of the Invention

The conventional procedures for developing new hybrids are well known and are described in the literature. The Encyclopaedia Britannica, Macropedia, has extensive sections on "Horticulture" and on "Plant Breeding." While the procedures are known and are widely practiced, their limitations are equally well recognized.

The plant breeder seeking to develop new hybrids will cross two parental lines, each composed of plants which are as homozygous (purebred) as possible. However, homozygosity is achieved slowly; plants must be selfed (inbred) for a number of generations to obtain an essentially pure breed. From eight to twelve generations is not uncommonly required to produce lines which are sufficiently homozygous so that, when crossed with other homozygous lines, a population of genetically uniform hybrids is obtained.

Moreover, only a relatively limited number of purebred lines are available, and this restricts both the possible number of crosses and the genetic diversity of the hybrids that can be made in any given year.

Still another limitation on the development of new hybrids is that the parent plants, being reproduced sexually, may develop undesirable genetic changes from generation to generation (genetic drift).

A further constraint on existing techniques of hybrid plant development is the fact that many plants produce only a limited number of seeds per plant. This is particularly troublesome because inbred parental lines exhibit low vigor, which manifests itself in low seed yields. Thus, even after superior hybrids have been developed, large scale production of hybrid seed for sale may not be possible for the next year or so.

The above conditions are epitomized in the development of canning and juice tomatoes. A fair number of purebred varieties are obtainable, but due to the limitations above, only a few new hybrids appear on the scene each year.

In addition to the existing problems in developing new hybrids, the current procedures used for the commercial production yields of high purity hybrid seeds in some plants, such as certain Brassicas (Brassicas include broccoli, cabbage, cauliflower, Chinese cabbage, and turnips), are widely recognized as having serious limitations, both in terms of cost and seed purity. The conventional methods all require the establishment of stable, nearly homozygous parental breeding lines by the repeated self-pollination (selfing) of each genotype. One, or both, of these inbred parental lines must be sib-incompatible (unable to readily pollinate with its siblings). Many of the Brassica genotypes are strongly self-incompatible (nearly incapable of pollinating itself), as a result, inbreeding to maintain the parental lines can only be effected by bud-pollination; a process wherein each flower bud is opened by hand and then pollinated from another flower on the same plant, again by hand.

These difficulties in maintaining the parental inbred breeding lines are reflected not only in high hybrid seed cost as a result of the labor cost, but, in practical effect, have required that the breeding lines be maintained in foreign low-labor-cost countries. Moreover, the parental lines are of necessity highly inbred, and the plants have low vigor, resulting in low $F_1$ hybrid seed yields.

In the production of brassica hybrid seeds by conventional methods, either of two approaches are used. In one method, the flowers in one parental line are emasculated by hand, and are fertilized with pollen from the crossing line. This method is labor intensive, and consequently expensive. A second approach, widely used on a commercial scale, avoids emasculation. Two inbred parental lines, one or both lines being sib-incompatible as well as self-incompatible are crossed by natural pollination. Hybrid seed is collected only from the sib-incompatible breeding line. In the usual case, where one inbred is sib-incompatible, a high ratio, typically 3:1, of the sib-incompatible inbred to the normal inbred is used, and only the seed produced from the sib-incompatible inbred is harvested for sale. Ideally, if both inbreds are sib-incompatible, a 1:1 ratio of the two can be used, and, due to the reciprocity rule of genetics, the entire seed crop can be harvested.

While the sib-incompatible technique for $F_1$ hybrid Brassica seed production avoids many of the difficulties of emasculation, a number of problems are encountered in practice. One such problem is that the large populations of inbred parent plants tend to undergo genetic drift since they are sexually reproduced.

An especially serious seed quality problem arises with the use of sib-incompatible hybrid techniques. If a "misnick" (poor timing between the two inbreds in their going into inflorescence) occurs, some sibbing takes place on the ostensibly sib-incompatible parent, giving rise to inbreds being present in the seed crop. This may result in the seed not being of sufficiently high purity to comply with present day labeling laws, which mandate that a hybrid seed contain at least 95% of the designated hybrid. Finally, the development of a sib-incompatible inbred normally requires at least about 10 years of inbreeding.

The techniques of vegetative propagation (asexual reproduction) have been used in the production of certain plants such as many fruits and nuts, artichokes, garlic, girasole, rhubarb, potatoes, and sweet potatoes. The conventional asexual techniques involve the use of asexual seed, specialized vegetative structures (runners, bulbs, roots, shoots), layering and cuttings, grafting and budding, or more recently, tissue culture. Tissue culture techniques have been widely developed for other purposes or for other plant species. See Encyclopaedia Britannica, Macropedia, "Tissue Culture," "Tissues and Fluids, Plant," "Horticulture," "Plant Breeding," "Fruits and Fruit Farming," and "Vegetables and Vegetable Farming," together with articles cited therein. State-of-the-art technology is reviewed in "Propagation of Higher Plants Through Tissue Culture," Proceedings of International Symposium, University of Tennessee, Knoxville, April 16-19, 1978 (Technical Information Center, U.S. Department of Energy, National Technical Information Service, U.S. Department of Commerce, Springfield, Virginia 22161, Conference T-80411); E. Thomas and M.R. Davey, "From Single Cells to Plants," (Wykenham Publ. 1975); and D.N. Butcher and D.S. Ingram, "Plant Tissue Culture," (Camelot Press, 1976).

Tissue culture techniques have been used to propagate certain hybrid plants themselves or parent plant lines (for example, orchids, carnations, broccoli, and asparagus). A pertinent literature reference is W.C. Anderson and J.B. Carstens, "Tissue Culture Propagation of Broccoli, Brassica oleracea (Italica Group) for use in

$F_1$ Hybrid Seed Production," <u>J. Am. Hort. Sci.</u>, 102(1), 69-73 (1977), and references cited therein. The use of tissue culture in asparagus is mentioned in the American Vegetable Grower, May 1978, pp. 8-9, and October 1978, pp. 8-9. Patent references incluide Kadkade U.S. 4,038,778, McCormick Brit. 1,387,821, Sibi U.S. 4,003,156, Routien U.S. 2,747,334, Tukacs U.S. 3,009,289, McDade U.S. 3,514,900, Corlett U.S. 3,683,550, Gudin U.S. 3,816,960, Stottlemeyer U.S. 3,821,864, Carlson U.S. 3,832,801, Patterson U.S. 3,861,079, Gudin U.S. 3,955,317, Boxus U.S. 3,972,146, Seibert U.S. 4,052,817, Kadkade U.S. 4,060,933, and Staba et al. U.S. 3,846,937. To the applicants' knowledge, none of these techniques provide a large scale method for the rapid development of new hybrids and for the rapid production of such hybrids on a commercial scale.

## 3. SUMMARY OF THE INVENTION

The principal object of this invention is to rapidly produce high purity hybrids and hybrid seeds in increased yield, thus providing a method for large scale commercial production and marketing. The invention is a process which combines the techniques of sexual reproduction (crossing) to develop new hybrids and hybrid seeds, and cloning (vegetative propagation) of the original parent plants to provide large scale replication of the original crosses.

The present invention is based in part on the recognition that the crossing of genetically different individual parent plants, one or both of which is heterozygous, can on occasion yield a population of $F_1$ hybrids that, while genetically different from each other, exhibit a high degree of phenotypic uniformity (i.e., similar physical characteristics).

A further recognition embodied in the invention is that hybrid seeds derived from clones of the respective individual parents similarly yield plant populations that are phenotypically similar, as well as genetically equivalent, to the original-parent-derived $F_1$ hybrids. Thus, where a cross of original individual parent plants (one or both of which is heterozygous) will yield phenotypically uniform original-parent-derived hybrids, crosses of clones of the parents also will usually yield phenotypically similar cloned-parent-derived hybrids.

These recognitions underlie the basic advantages of the invention. Because one or both of the original parent plants can be heterozygous, the parent (or parents) can be vigorous hybrids instead of low-vigor inbreds. Consequently, the hybrid seed yield will be high.

Further, because the parental "lines" are numerically limitless, the plant breeder has available breeding "lines" of limitless genetic diversity. As a consequence, hybrids can be produced with characteristics that are equal to, or superior to, hybrids that are derived from conventional crossing of highly inbred homozygous parents. Moreover, this is accomplished with unprecedented economy.

In accordance with one aspect of the invention, a cross is made from two individual parent plants, either or both of which may be heterozygous. The individual original parent plants are prepared for vegetative propagation, or cloning, using tissue culture techniques. Hybrid seeds from the crosses are planted, and if these original- parent-derived hybrid plants exhibit phenotypic uniformity, clones of the respective individual parent plants are propagated. If not, the germplasm may be destroyed. Crossing the respective clones then yields seeds of cloned-parent-derived hybrids that are genetically equivalent to the original-parent-derived hybrids, and that are themselves phenotypically uniform.

In accordance with another aspect of the invention, a large number of experimental crosses (top crosses) are made between individual parent plants which differ genotypically, without limitations on the heterozygosity or homozygosity of the parents. The individual original parent plants used in the crosses are vegetatively propagated, or cloned, using tissue culture techniques. In field trials, the hybrids resulting from the large number of experimental crosses are evaluated, and a limited (small) number of optimum (i.e., best performing) original-parent-derived hybrids are selected for development. Those selected express a combination of phenotypic uniformity within the hybrid, and possess the desired hybrid characteristics. Once the selection has been made, the original individual parents of each of the selected optimum original-parent-derived hybrids are vegetatively propagated to produce clones of the individual parents of each hybrid. Then, the two cloned parent lines for each hybrid are crossed, on a larger scale, to produce seeds. Seeds produced in this manner will yield a population of cloned-parent-derived hybrid plants that are phenotypically similar to (i.e., genetically equivalent to) the optimum experimental original-parent-derived hybrids that were selected for commercialization.

This process allows the plant breeder to select as parents optimum, superior, plants or sports which exhibit desirable genetic characteristics, e.g., plant vigor, growth habit, yield, disease resistance, resistance to water or salt stress, fruit or juice quality, etc. Thus, instead of being limited to a relatively small number of low vigor homozygous parental lines, the breeder can utilize any plant, irrespective of its degree of heterozygosity, in making the crosses. As a result, new experimental hybrids are developed with unprecedented genetic diversity.

Once a large number of top crosses has been made, each resultant hybrid population which demonstrates phenotypic uniformity and desirable hybrid traits is then exploited for hybrid seed production by utilizing the cloning technique applied to their respective original parent plants. Crossing the cloned parent plants produces cloned-parent-derived hybrid seed on a large scale that is genetically equivalent to the selected optimum original-parent-derived hybrid seed.

When the process is used for the commercial production of high purity Brassica seeds an unexpected advantage is obtained when either one or both selected parents is maximally self-incompatible. Cloning a parent plant that exhibits a maximum degree of self-incompatibility results in a parental line consisting of plants which are effectively sib-incompatible. To demonstrate, if maximally self-incompatible plants are cloned, the resulting clones, each being "self," will not readily pollinate with each other; accordingly, the clones of the parent plant line are effectively sib-incompatible as well as self-incompatible.

Consequently, when one cloned parental line is naturally cross-pollinated with another parental line, only pollen from the latter can fertilize the first line. As a result, seed from the first line is of a uniformly high hybrid purity, with little or no likelihood of self-fertilization (inbreeding). If both of the parental lines are cloned parents, each selected for being maximally self-imcompatible, then natural cross pollination of the two lines gives a seed crop obtainable from both lines.

The cloning technique has a major advantage over pre-existing methods of making hybrid seed. It eliminiates the need for the elaborate and expensive procedures for establishing and maintaining self-incompatible, nearly homozygous, parental breeding lines, which heretofore have required laborious manual bud pollination. Sib-incompatibility of the clones should be a stronger reaction that sib-incompatibility of inbreds because self incompatibility is a stronger reaction. By cloning optimal parents, the clones retain the optimum genetic characteristics of the parents. Further, when the cloning is effected according to the optimum practice of the present invention, the original-parent-derived hybrids are genetically equivalent to the cloned-parent-derived hybrids.

## 4. DESCRIPTION OF THE INVENTION

### 4.1 Development of Breeding Strategy

As the initial step in development and production of new hybrids, a breading strategy is formulated. Broadly stated, the goal of this breeding strategy is to cross individual plants to produce hybrids which combine desirable traits of each of the parental lines. The particular traits are, of course, defined by the commercial requirements of the market (e.g., disease resistance, yield, fruit or seed quality for edible plants, appearance for ornamentals, etc.) and the farming practice to be used.

Heretofore, the plant breeder has been required to be judicious in his selection of parental lines for crossing to produce hybrids because essentially homozygous parental lines for many plants are relatively few in number. Further constraints are the relatively high cost of multiple selfings and field trial selections needed to develop the homozygous parent lines, and of crossings and trials necessary to verify the quality of the hybrids. According to the present invention, however the breeder is offered an almost limitless number of parental breeding "lines," as each "line" may comprise as little as one plant which itself may be--and desirably is--a hybrid. No longer does the breeder work with breeding lines; he now can use a single plant as a breeding "line." Accordingly, the breeding strategy may take advantage of any desirable trait found in any single plant, and this plant may be crossed to any other plant to combine the desirable traits.

Stated differently, no longer is the breeder limited to a few homozygous parental lines. An outstanding individual plant, whether or not homozygous, can be crossed to a number of other plants (again, irrespective of their homozygosity) and any of the resulting hybrids which prove of value and which exhibit phenotypic uniformity can be developed and commercialized rapidly.

Moreover, the large number of different hybrids that can be developed in a single growing season permits optimum selection of superior hybrids from among them.

### 4.2 Selection of Individual Parent Plant

As the second step in the procedure, individual parent plants are selected. As previously mentioned, plants with desirable traits are selected with no limitations imposed on the heterozygosity or homozygosity of the plant.

### 4.3 Crossing of Individual Original Parent Plants

From the original parental lines--and, again, a "line" may be even a single heterozygous plant--a large number of top crosses are made according to the desired breeding strategy. Because the present technique permits the almost-endless replication of original parent plants, a corresponding large number of top crosses can be made. Thus, any individual plant that exhibits a desirable characteristic may be used to provide pollen to fertilize other plants, or to be fertilized by pollen from another plant, regardless of its homozygosity or heterozygosity.

Accordingly, individual plants that exhibit desirable traits such as high vigor, growth habit, yield, disease resistance, water stress resistance, salt stress resistance, trueness to type, self-incompatibility, or any other objectively or subjectively desired characteristic may be used for crossing. If the resulting cross exhibits the desired combination of characteristics, additional plant material (germplasm) is available for large scale duplication of the cross by cloning the parent plants. If not, the hybrid as well as its parents can be destroyed with minimal cost of time or expense.

In keeping with a preferred embodiment, a population of, say, five to twenty individual plants from within a line is chosen for crossing. This allows one line to be crossed onto several other lines, or permits several lines to be crossed onto the one. As will be apparent below, quite frequently one line contributes desirable genetic characteristics to several different hybrids.

Crossing of the individual parent plants is by conventional hybridization techniques for the species selected. Pollen from one designated individual plant (the male, or pollen donor) is applied to the pistils of a female, which is usually an emasculated plant. Either manual pollination from the anthers of one plant onto the pistils of another, or mechanical techniques of pollen collection and fertilization, may be employed, depending upon the particular type of plant.

4.4  Obtaining Clonable Explant Tissue

After designating the individual parent plants, and usually although not necessarily before crossing, suitable explant tissue is obtained from each of the individual original parent plants. The explant is to form the basis of vegetative propagation of the individual original parent plant, preferably by tissue culture techniques.

Obtaining explant tissue depends largely upon the particular plant. Since tissue culture propagation is preferably employed for the cloning, that part of an individual plant is selected which is capable of being developed under tissue culture conditions to increase in number and then form an entire plantlet that can be transplanted to soil and ultimately to the field. Such plant parts as axilary buds or shoot apices (e.g., for tomato plants and cabbage), curds (e.g., for cauliflower), root and stem tips, or other plant parts that can be propagated without the risk of detrimental genetic change, may be used.

Either the original parent plant itself, or normal horticulturally or vegetatively propagated counterparts, is used for obtaining clonable explant tissue for culture.

The first step in obtaining explant tissue is to assure that the plant itself is insect, disease, and fungus free. Pesticide treatment, advantageously in a greenhouse environment, may be utilized, with careful precautions being taken to assure, so far as possible, that contaminant micro-organisms are excluded. Thus, insecticides and fungicides are applied prophylactically, or the plants may be grown in isolation.

A few days prior to obtaining explant tissue, the plant and plant area are prepared for excision to remove the explant. The plants are desirably sprayed with a fungicide solution, which is also administered to the soil surrounding each plant.

Immediately prior to obtaining the explants, the plant tissue area is sterilized with an appropriate antiseptic or aseptic agent, advantageously a 70% ethanol-water solution. Sterile scalpels, forceps, and gloves are used for the actual excision and for the transfer of explant tissue to the tissue culture facility.

Desirably, several explants are taken from each plant. While the tissue culture techniques that are presently available result in high survival rates, it is advantageous to insure the availability of germplasm of an individual plant by obtaining sufficient explants to protect against chance mortality.

From this point onward, aseptic laboratory conditions must be maintained for the cloning. The nutrient medium for propagating plants must itself be sterile, as the medium will in most instances favor microorganism growth. Accordingly, the laboratory area is maintained sterile, and all transfers of vegetatively developed shoots or other material is effected in a germ-free environment.

### 4.5 Optional Storage of Explants or Shoots

Because at least one, and usually at least two, generations are required for the development and commercial production of new hybrids using the present methods, it is necessary to maintain the germplasm of each parental line intact for a corresponding length of time before actual commercial production. Thus, the explant tissue, or other forms of tissue cultures such as shoots developed from explant tissue, must be preserved genetically intact during this period.

In the case of perennials, the plant itself may be grown in isolation for several years, and explants removed at any time. This is the simplest procedure, and is optimum practice when it can be effected.

For plants having only one or two growing seasons, either of two alternate approaches can be taken.

First, an explant or cutting can be vegetatively propagated, and the propagation repeated every several weeks, or as frequently as necessary, in order to insure a supply of shoots when it becomes necessary to produce more hybrids from clones of the original parents.

A second procedure, which has not been sufficiently developed at present, is to utilize cryogenic techniques to store and preserve either explants or shoots. Ideally, liquid nitrogen temperature should permit such storage for months or even years. Others have utilized low temperature storage, e.g. Seibert U.S. 4,052,817 with various disclosed cryoprotectants added to prevent tissue damage. Siebert also suggests storage below minus 70°C after controlled freezing and subsequent thawing.

4.6 Cloning Explant Tissue

The explants or cuttings removed from individual parent plants are then propagated vegetatively, or cloned, under tissue culture conditions so as to produce a number of plantlets, each genetically similar to the patent genotype.

In substance, a part of a plant is caused to grow or multiply, or both, in vitro under sterile conditions, in a preferably synthetic nutrient medium. The composition of such medium includes: inorganic ions necessary to maintain normal fluid balance and to act with certain enzymes; energy sources such as sucrose, which also supply a major source of carbon atoms for forming cell constituents; nitrogen-containing compounds such as amino acids which are the basic building blocks for proteins of the cell;

essential vitamins; certain hormones such as auxins and/or cytokinins for growth of particular plant structure and the like. Nutrient media are described, for example, in standard references by Murashige and Skoog, Skoog, Heller, Knop, Gamborg, White and Street.

An agar gel medium is advantageously employed for in vitro cloning, especially when using organogenic methods. Gel propagation methods as well as the less-desirable liquid suspension (shake) culture technique are described, for example, in E. Thomas and M.R. Davey, "From Single Cells to Plants," (Wykenham Pupl. 1975), and in D.N. Butcher and D.S. Ingram, "Plant Tissue Culture," (Camelot Press, 1976).

Depending on the particular plant, different in vitro techniques have been found to be desirable. For tomatoes, as set forth more fully below, a three stage technique is employed; in the first stage, or initiation, shoot cultures are established from axillary buds in a basal medium containing a low concentration of an auxin; in the second stage, multiplication, the nutrient medium contains a higher concentration of auxin so as to permit the growth and development of the shoots which are generated in the initiation stage; in the third stage, root development, a higher auxin content is utilized. A similar three stage procedure has been found desirable for broccoli (see W.C. Anderson and J.B. Carstens, "Tissue Culture Propagation of Broccoli, Brassica oleracea (Italica Group), for Use in Fl Hybrid Seed Production," J. Amer. Soc.Hort.Sci., 102(1), 69-73 (1977). Other tissue culture procedures are either known or may be developed for other plants.

In a typical protocol of the present invention, represented by tissue cloning techniques developed especially for tomatoes, a three stage procedure is utilized. Approximately 10-20 axillary buds, aseptically removed from each parent plant, are grown in an initiation medium for about 4 to 5 weeks. Each bud forms a shoot under these conditions.

At the end of this time, the shoots are removed from the agar medium, and axillary buds from the shoots are again carefully removed, with the aid of a stereomicroscope. The buds are then transferred to a multiplication medium, where, within a month or so, each bud grows into a shoot possessing approximately three or more additional buds. The buds are then separated, and transferred either to a third medium where, at a high auxin content, root development proceeds and a complete plant is formed, or to another cycle on the multiplication medium for further increase in number.

Finally, when sufficient plants are available from culture, they are separated and transferred to a greenhouse for development into normal-appearing plants which are in fact clones of the original parent plants.

In the preferred approach of the present invention for cabbage, isolated shoot apices along with one or two leaf primordia, are excised from the selected plants and are treated in a single stage tissue culture protocol. This stage combines plant shoot establishment, multiplication and rooting. (A multiple stage protocol may alternatively be followed--i.e., culture establishment, multiplication and rooting--but is not essential.)

For cabbage, the preferred protocol for vegetative propagation is to culture shoots from isolated apices. This has been found to be effected best in a single stage agar-based nutrient medium that contains an auxin (e.g., 0.25-5.0 mg/l) and a cytokinin (e.g., 1-10 mg/l).

The shoots thus produced may be multiplied indefinitely or, if desired, can be stored. Cryogenic storage techniques have not yet been perfected for cabbage, but similar techniques have been used for other plant species; see Seibert U.S. 4,052,817.

After approximately three or four weeks, sufficient development of the shoots takes place to permit further propagation by multiplication in vitro, or the propagated shoots (plantlets) may be transplanted to greenhouse conditions for ultimate growth in the field to maturity. A hardening off treatment, consisting of maintaining the plantlets in a sterile (peat/vermiculite mixture) potting medium under conditions of mist and high humidity, prepares the plantlets for transplanting.

In the preferred approach of the present invention for cauliflower, cauliflower curds are excised from the selected inbred plants and are treated in a three-stage tissue culture protocol. These stages optimize plant shoot establishment, multiplication and rooting, by controlling the content of plant hormones in each of the stages.

For cauliflower, the first stage in the protocol of vegetative propagation is to establish a shoot culture from meristematic tissue of cauliflower curds. This has been found to be effected best in an agar-based nutrient "medium that is comparitively high in auxin (e.g., 2.5-10 mg/1) but low (e.g., 0.5-2.5 mg/1) in cytokinin. Thus, curds placed in the first stage propagation medium develop individual shoots.

After a period of growth in the first stage medium approximating two or three weeks, the shoots can be isolated for multiplication in the second stage.

When it is desired to multiply the shoots in the second stage, shoot explants are placed in a second nutrient medium. This contains a reduced content of auxin (e.g., 0.5-2.5 mg/l) and a much higher content (e.g., 2.5-10 mg/l) of cytokinin, the latter of which is conducive to multiplication or proliferation. This medium contains a phosphate, and with the composition described below gives a multiplication rate of about 13 shoots every two weeks or so.

The shoots produced in the second stage can be multiplied indefinitely or, if desired, can be stored. Cryogenic storage techniques have not yet been perfected for cauliflower, but similar techniques have been used for other plant species; see Seibert U.S. 4,052,817.

The shoots from the second stage lack adequate root development, and for this reason are placed in a third, or rooting, stage containing low auxin (e.g., 0.5-2.5 mg/l) and very low cytokinin (e.g., 0.001-0.5 mg/l).

After approximately two or three weeks, sufficient root development is observed to permit the propagated shoots to be transplanted for ultimate growth in the field maturity. A hardening off treatment, consisting of maintaining the plantlets in a sterile (peat/vermiculite mixture) potting medium under conditions of mist and high humidity, prepares the plantlets for transplanting.

## 4.7   Selecting Optimum Hybrids

When the original experimental hybrids derived from the individual parent top crosses have grown to maturity, it is possible to select from among the hybrids a limited, small, number of optimum hybrids that exhibit the desired characteristics.

First, the population (typically from 25 to 200 in number) of hybrid plants from each individual cross is examined for uniformity of phenotype.  Those crosses whose populations show a high degree of phenotypic uniformity within the individual cross are selected for further evaluation.  Those crosses that show significant phenotypic segregation (non-uniformity) are discarded.

Those hybrids that have passed the above test are now examined for hybrid vigor, disease resistance, yield, plant and fruit quality, and other characteristics that are desired in cultivars having maximum commercial utility.

In the case of tomatoes, tomato shape and the quality of the fruit and fruit juices are the prime considerations.  Other factors, such as wilt resistance, salt tolerance, and the like also enter into consideration in selecting optimum (originalparent-derived) hybrids.

An additional fact, of particular importance when the hybrids are to be harvested mechanically, is concentration maturity, that is, the extent to which all members of the population mature at approximately the same time. This becomes important when there is no opportunity to manually select or reject fruit for harvesting.

Once the small number of optimum (original-parent-derived) hybrids has been selected from among the large number of experimental hybrids, vegetative propagation of the individual original parent plants of each of the selected optimum hybrids is commenced. All other germplasm of un-selected parent plants--whether the plant itself, cuttings, explants, or shoots--may then be discarded.

Clones of the individual original parent plants of each of the selected optimum experimental (original-parent-derived) hybrids are then propagated and multiplied to provide a large number of clones, one set of clones corresponding to each of the two original parent plants of each selected hybrid. The procedures of propagation and multiplication were described earlier above, and are specified in more detail in the examples.

### 4.8 Crossing Cloned Parental Lines

Having vegetatively propagated each of the individual original parent plants of each of the selected optimum experimental original-parent-derived hybrids, it is now possible to cross the cloned parent plants to produce seeds of cloned-parent-derived hybrids. These cloned-parent-derived hybrids are genetically equivalent to the original hybrids inasmuch as clones of the original parents are genetically equivalent to the original parents themselves.

The procedure used to cross the cloned parent plants to produce seeds of coned-parent-derived hybrids parallels that of crossing the original parent plants to produce experimental original-parent-derived hybrids. The main difference, of course, is that many clones are now available corresponding to the respective original parent plants.

For optimum practice, pollen from clones of the original parent plant that had been used to provide pollen for the crossing of individual original-parent-derived hybrids is utilized in the crossing of cloned parental lines. Correspondingly, clones of the plant that had been the female member (seed parent) of the pair are used as females in the crossing of the clones.

Procedurally, and depending primarily on the type of plant involved, the larger-scale production of hybrid seed from coned parents is accomplished in the same manner as was used for production of original-parent-derived hybrids in the first instance. The scale, of course, is much greater, depending on the amount of hybrid seed that is required.

4.9  Verification by Field Trials

Plants within an individual hybrid population must have an acceptably uniform phenotype to be of significant commercial interest. (Genetic uniformity, of course, is not achievable unless both parents are homozygous.) Phenotypic similarity manifests itself in gross visible characteristics of the plant population, but other criteria may also be present. In processing of hybrid tomatoes and other mechanically harvested plants, one important measure of phenotype uniformity is essentially simultaneous maturation (concentration maturity) of all plants within the hybrid population.

When the procedure of the present invention is followed, in theory at least all plants within a population of cloned-parent-derived hybrids are phenotypically similar to each other and are genetically equivalent to the original-parent-derived hybrid. Since only those original-parent-derived hybrids were selected that exhibited phenotypic uniformity, it can be expected that phenotypic similarity will exist among the cloned-parent-derived hybrids. In practice, however, in view of the large expenditures involved in producing and then using hybrid seed, it is economically desirable to verify phenotype uniformity by field trials.

Field trials (pilot production) are conducted on a semi-commercial scale to verify the genetic equivalence between cloned-parent-derived hybrids and original-parent-derived hybrids, as well as to verify the phenotypic uniformity of plants within the population of cloned-parent-derived hybrids.

Field trials to verify the equivalences are conducted using hybrid seeds obtained by crossing clones of the respective original parent plants, on a sufficient scale to provide the quantity of seeds necessary for field trials. Success of the field trials verifies success of the combined crossing and cloning procedure of the present invention.

At the field trial level, performance of the selected hybrids can be monitored, and any problems can be detected before full scale commercial production begins. If the problems are sufficienty serious, then the hybrid strain can be discarded along with its parental material.

## 4.10  Commercial Production

Once cloned-parent-derived hybrids have been grown to maturity and harvested to provide for pre-commercial verification in the field trials, full scale commercial production of hybrid seeds can begin.

The procedures of cloning the individual original parent plants by vegetative propagation that were described earlier are repeated to obtain additional cloned parent plants. The clones of the respective original parents are then crossed to produce large quantities of hybrid seed for sale.

In succeeding productions, additional clones of the individual original parent plants are propagated to provide germplasm for producing the next generation of cloned parents for crossing to produce seeds of cloned-parent-derived hybrids. These similarly are genetically equivalent both to the prior generation of cloned-parent-derived hybrids and to the original parent-derived hybrids.

## 4.11  Applications

The technique of the present invention is applicable to a wide variety of monoecious crops. Provided only that parent plants can be cloned, and that the germplasm can be maintained over a number of generations, any plant which is susceptible to hybridization can be used in the production of new hybrids.

Vegetable crops are particularly valuable candidates for this method of new hybrid production. The high cost of seed, the frequent low seed yield per plant, quickly shifting disease  and pest patterns, and the economic value of superior cultivars favor the rapid development and commercial production of new hybrids. Tomatoes are especially important, and are exemplified herein.

Other garden crops such as melons likewise may be treated according to the present invention. The potential improvement is great for each of these agricultural species, and there is a demands for new and better varieties of each.

Other vegetable, particularly the <u>Brassica</u> species of cauliflower, cabbage, and the like, similarly may be improved by the formation of new hybrids and their rapid development according to the invention. In these cases an unexpected advantage is obtained when either one or both selected parents is maximally self-incompatible. Cloning a maximally self-incompatible parent plant results in a parental line that is effectively sib as well as self-incompatible. Thus the conventional necessity of bud-pollination for inbreeding maximally self-incompatible plants is eliminated.

Economically, cereals do not appear to be a prime candidate for the invention herein, although the process is applicable. For some plants such as rice, there may be too few seeds per plant for the system to become economic, and for other cererals the value of hybrids as compared with preexisting plant varieties may not be large.

In substance, therefore, the technique of the invention provides a method of rapidly developing and commercially producing new, commercially useful, hybrids from the crossing of individual plants that need not be homozygous. It comprises the steps of (a) crossing pairs of selected individual original parent plants to produce experimental original-parent-derived hybrids, (b) selecting optimum original-parent-derived hybrids that exhibit both phenotypic uniformity and useful hybrid characteristics, (c) cloning, by vegetative propagation, each of the individual original parent plants of each of the selected experimental original-parent-derived hybrids so as to obtain cloned parent plants, and (d) crossing the cloned parent plants to provide seeds for cloned-parent-derived hybrids that are genetically equivalent to the selected original-parent-derived hybrids.

### 4.12   Example 1.   Tomatoes

In this Example, tomatoes were crossed to develop four new hybrid varieties. Their respective parents were clonally propagated, and the crosses of the clones were made. Each of the four hybrid varieties exhibited a population of uniform phenotypes and demonstrated yields, maturity concentration, fruit quality factors, and plant habits equal or superior to the best presently (1979) known varieties.

### 4.121   Selection of Individual Parent Plants

Initially, sixty lines and/or cultivars of tomatoes were chosen, each differing genotypically from any other. Many of the lines were heterozygous, and in some instances were $F_1$ hybrids. Seeds from these lines were planted in greenhouse flats to establish a population of from 100 seedlings, from which a small number of individual plants (e.g., one to ten) of each were selected. Each of these individual plants was then designated, by physical tagging, for use as a parent in the subsequent crossing. 144 individual parent plants were so designated, and were chosen as genotypes for hybridization.

4.122 Crossing of Individual Parent Plants

A large number of crosses were made from the selected 144 individual parent plants. The objectives of the crossing strategy were to obtain tomatoes having the desired characteristics of fruit shape and quality. Thirteen major classifications based on fruit morphology were utilized; e.g., shape being square round to square round, square round to blocky, round to round, elongated to elongated, blocky to round, etc.

The pollen from an individual designated male plant within a line was then crossed to several designated individual (emasculated) females. Conventional manual pollination techniques were employed, using pollen from multiple flowers in one designated individual plant to fertilize multiple emasculated flowers on a designated female plant.

The resulting tomatoes from each specific cross were harvested, and the seeds were collected.

Seeds from each specific cross were planted in field trials for comparison testing; approximately fifty-to-one hundred plants from each cross were thus planted. Cultivation was by conventional farming techniques typical for the area.

At the appropriate stage of maturity, data were collected with respect to pathogen resistance, vigor, fruit shape, number of fruit per plant, and other factors identified below.

It was ultimately found that, of the original large number of crosses, four hybrids were outstanding. These were:

(a) 6290-5 x 6269-2; Fusarium Race II Resistant Early Round, obtained by top-crossing a single plant out of a breeding line obtained in the $F_5$ from the University of California (Davis) onto a single plant from GS 12 Hybrid (Goldsmith).

(b) 6240 x 6238-2; Fusarium Race II Resistant Medium Early Square Round, a single plant out of the variety of U.C. 82A crossed to a single plant of Keystone Exp. 9976.

(c) 6274-2 x 6250-2; Fusarium Race II Resistant Medium Late Square Round. This was obtained from a single plant out of the $F_1$ of the cross between Keystone Exp. 9976 and a U.C. (Davis) breeding line, crossed to a single plant out of an $F_7$ breeding line (Keystone) derived from a long fruited 198 type crossed to Keystone super concentrated material.

(d) 6276-2 x 6250-2; Fusarium Race II Resistant Medium Maturity Square Round. A single plant out of the $F_1$ of the cross between U.C. (Davis) breeding line and a Keystone breeding line out of an unknown parentage crossed m Race II Resistant Medium Maturity Square Round. A single plant out of the $F_1$ of the cross between U.C. (Davis) breeding line and a Keystone breeding line out of an unknown parentage crossed to the same $F_7$ breeding line used in the immediately preceding cross.

A semi-quantitative inspection of tomatoes from the various crosses is given below.

# HYBRID PROCESSING TOMATOES

## FRUIT QUALITY

| RATING | VARIETY | SHAPE OF FRUIT | VISCOSITY | pH | SOLIDS | SHAKING TEST** | | | | |
|--------|---------|----------------|-----------|-----|--------|----------------|--------|-------|-------------------|------------------|
| | | | | | | NO STEM | W/ STEM | GREEN | SHATTERED & ROTTEN | TOTAL RED FRUIT |
| XX | 6290-5 x 6269-2 | Round | 99.4 | 4.23 | 6.24 | 21-1/2 | 35 | 9-1/2 | 2-1/2 | 56-1/2 |
| X | 6240 x 6238-2 | Square/ Round | 236.3 | 4.36 | 4.90 | 83 | 7 | 21 | 1/2 | 90 |
| XX | 6274-2 x 6250-2 | Square/ Round | 292.7 | 4.41 | 5.26 | 33-1/4 | 26-1/4 | 59 1/2 | 2-1/8 | 59-1/2 |
| XX | 6276-2 x 6250-2 | Square/ Round | 231.0 | 4.27 | 5.08 | 23-3/4 | 35-3/4 | 47 | 2-1/2 | 58-7/8 |
| | 7879 K3* | Round | 138.5 | 4.30 | 5.3 | 32-5/8 | 29-1/2 | 16-3/4 | 6 | 62 |
| | GS-12* | Round | 224.0 | 4.38 | 5.78 | 12-1/2 | 18-3/4 | 3/4 | 2-1/2 | 31-1/4 |
| | GS-9* | Plum | 311.7 | 4.44 | 5.75 | 56-1/2 | 4-3/4 | 4 | 7-3/8 | 61-1/4 |

* These results represent data from several replications remote from the experimentals and thus are indicative only.

** This is an empirical simulation of mechanical harvesting. "No stem" and "total red fruit" are desirably maximized.

## 4.123 Obtaining Clonable Explant Tissue

The designated individual parent plants, while in the seedling stage in the greenhouse, were prepared for obtaining clonable explant tissue. From one to four cuttings of each of the parent plants were obtained, without surface sterilization, and were prepared for protected shipment to the tissue culture facility. The cuttings were placed in culture tubes containing an agar media similar to that described below for clonal propagation, except that (a) vitamins and sucrose were omitted, (b) the auxin was indolebutyric acid 1 mg/1, and (c) the agar concentration was increased 1% to withstand shipping.

Upon arrival at the tissue culture facility a week later, the cuttings were removed from the culture tubes and placed in moist sterile potting soil (1/1 peat/perlite) in a temperature controlled greenhouse. Plants grown from these cuttings were those actually used as a source of the explant tissue for culture establishment.

Several precautions were taken in growing the plants in order to obtain sterile, disease-free plants for subsequent tissue culture. To keep the plants free from insects at all times, pesticides were employed; alternatively, if an insect-free greenhouse is available, the plants can be grown in isolation. Fungicides were prophylactically applied where warranted.

Approximately two days prior to taking explant tissue, the plants were sprayed with a fungicide (Physan 20, Consan Pacific, Inc., Whittier, California, 2.6 mg/1), and 0.5-1.0 mg/1 of the same solution was applied to the soil surrounding each plant. This was repeated one day before taking explant tissue.

The procedure selected for clonal propagation was multiplication of axillary buds. Desirably, about ten buds were taken from each plant. To collect these buds, the plant tissue area was first sprayed with a 70% ethanol solution, and the bud area was excised with a sterile scalpel. The tissue was thereafter handled with sterile forceps or gloves washed with 70% ethanol, and was placed in culture tubes containing sterile water.

Sterilization of the explants was effected by treatment with a sodium hypochlorite solution (20% Chlorox) containing a surfactant (Tween 20, 2 ml/l). The solution and tissue were agitated for about 30 seconds, and then placed in a vacuum dessicator; 30 cm mercury vacuum was applied for 15 minutes to insure complete contact with the sodium hypochlorite.

After vacuum treatment, the solution was decanted and rinsed three times with sterile distilled water, agitating between each rinse.

Damaged tissue was then aseptically removed, and the axillary buds were placed on an sterile agar medium described below for culture initiation.

### 4.124 Optional Storage of Explants or Shoots

Two different methods have been employed to maintain the germplasm of the individual parent genotypes over a period of several generations.

In the first, a small bank of greenhouse plants was established from the cuttings of each individual parent plant. All plants were kept insect and disease free.

In the second method, a bank of twenty to thirty cultures of each genotype was maintained by serial transfer of shoots, as described in the section on cloning, below.

With either procedure, once it was ascertained that seven individual parent genotypes were to be replicated by clonal, or vegetative, propagation, the other 137 genotypes were discarded.

### 4.125 Cloning Explant Tissue

Cloning was effected in three stages--initiation, multiplication, root development--each under selected tissue growth conditions. First, cultures were established on an initiation nutrient medium containing a low concentration of indoleacetic acid (IAA), and were permitted to grow for four to five weeks. A single shoot developed from each bud.

Axillary buds excised from each of the shoots derived from the initiation stage were then transferred to the second stage, or subculture, for multiplication. This was effected in a nutrient medium which contained a higher concentration of IAA, and permitted the growth and development of shoots from each of the axillary buds obtained in the initiation stage. Multiplication was achieved by growing the axillary bud into a fully developed shoot within the culture tube, and thereafter excising from that shoot each of the axillary buds which developed on the shoot. The individual buds were then placed in separate tubes to produce a new shoot from each bud. Multiplication can be repeated every four weeks or so as needed.

When sufficient shoots (clones) were available from the multiplication stage, the third culture stage was employed. This utilized a higher IAA content in the nutrient to develop the roots on each shoot.

The following was the basal nutrient gel medium composition:

Medium Composition for In Vitro Culture of Tomato

| Constitutents | mg/l |
|---|---|
| Inorganic Salts | |
| Murashiage and Skoog Salt Formulation | a/ |
| $NaH_2PO_4 \cdot H_2O$ | 170.00 |
| Iron Complex b/ | |
| $FeSO_4 \cdot 7H_2O$ | 27.85 |
| $Na_2EDTA$ | 37.25 |
| Vitamins | |
| Nicotinic Acid | 0.50 |
| Pyridoxine.HCl | 0.10 |
| Thiamine.HCl | 0.50 |
| Hormones | |
| 3-indoleactic acid (IAA; Auxin) | 0.03-3.00 |
| $N^6$-furfuryladenine (Kinetin) | 0.03 |
| Ancillary Organics | |
| Clycine | 3.00 |
| Inositol | 100.00 |
| Sucrose | 30,000.00 |
| Agar (Sigma Grade IV) | 7000.00c/ |

a/  T. Murashige and F. Skoog, "A revised medium for the rapid growth and bioassays with tobacco tissue culture," Physiol. Plant., 15, 473-497 (1962).

b/  Maintained as fresh stock solution.

c/  pH at 5.7 prior to autoclaving 15 min. at 121°C. at 16-18 psi.  20 ml media/culture tube 25 x 150 mm.

In more detail, the sterile axillary buds excised from the individual designated parent plants (or indirectly from their cuttings) were first transferred to an initiation medium comprising the basal medium in the table above, containing 0.03 mg/l of indoleacetic acid (IAA) as the auxin, and allowed to grow for about four to five weeks prior to subculture.

After this growing period, the shoots were individually removed from their cultured tubes and, under sterile conditions, the axillary buds were then carefully excised from each shoot.

These buds were then transferred to a multiplication medium, having the composition set forth above, with the exception that the IAA content was 0.3 mg/l. In four weeks, each axillary bud developed into a shoot having approximately two to seven axillary buds, although this is highly variable.

The multiplication cycle was repeated every four weeks or so, in each case removing the developed shoot, excising the axillary buds, and repeating the initiation and multiplication stages for additional shoot proliferation. When the original-parent-derived hybrids have grown sufficiently to permit selection of a small number of optimum hybrids, as described previously, a determination was made as to which individual original parent plants yielded optimum hybrids. In the Example described herein, seven such individual plants were identified. Germplasm of the seven was maintained, while shoots from the remaining 137 plants were discarded. Germplasm of these seven specific parents was retained for further multiplication.

When sufficient numbers of axillary buds from the seven selected original parent plants were available, the respective shoots were transferred to a third medium containing 3.0 mg/l IAA for root development. After two weeks, the cultured shoots showed good root development, and the plantlets were ready for transfer from tissue culture conditions to the greenhouse.

During the initiation and during the multiplication stages, temperatures of 25° ± 3°C., and light intensity from cool white fluorescent bulbs of 3500 lux was maintained, with a photoperiod of 16 hours. For root development, a light intensity of 6,000-10,000 lux was used.

The conditions above are apparently genetically conservative, in that virtually no phenotypic variants were observed. The conditions permit germplasm to be maintained as a bank by repeated serial transfer in the multiplication medium, and permit crossing of cloned plants for several years in succession.

### 4.126  Planting of Plantlets

The plantlets, or rooted shoots, obtained from the final stage of clonal tissue culture were then carefully planted in controlled growth rooms. For each selected parent genotype, a greenhouse ground bed was thus established containing 48 plants cloned by the above procedure from each of the original seven parent plants.

The plantlets were first removed from their culture tubes, agar was washed from the roots, and then planted approximately 10-15 mm below the root collar in moist peat/vermiculite in seedling trays (2 inch by 2 inch cells). Trays were placed in plastic tubes to control moisture loss, and were maintained in growth rooms at 28°C. and 16 hour photoperiods of 1500-2000 lux from cool white fluorescent bulbs.

After two days in the growth rooms, the plastic tubes were opened to harden off the plants, and the tubes were fully removed three days later. After an additional three days in the growth room, the plants were transferred to seedling trays in a greenhouse in full sunlight.

Following one week of growth in the greenhouse seedling trays, the plants were transferred to greenhouse ground beds to produce cloned parental lines of each of the respective individual parent plants.

4.127    Crossing Original Parent Plants

As indicated previously, top crosses were made utilizing the 144 individual original parent plants.

Conventional tomato crossing techniques were utilized. For each cross, the flowers on one plant were emasculated by removal of the anthers before the pollen grains had developed. Fertilication was effected by collecting pollen from multiple flowers on the other (male) plant and manually distributing it onto the pistils of the emasculated (female) member of the pair.

The female plant was then permitted to develop tomatoes to maturity. Tomatoes from each female were collected, and the seeds thus obtained constituted the hybrid seeds for planting in field tests.

In a refinement of the above procedure, several individual parents from within each parental line are initially chosen. The most vigorous parent from each line is then crossed to the most vigorous parent of another line.

Hybrid seeds thus obtained are subjected to a field test procedure, as described previously, to ascertain whether tye hybrids, as a population, possess the desired genetic characteristics.

### 4.128 Crossing Cloned Parental Lines

The procedure of crossing cloned parent plants to produce seeds of cloned-parent-derived hybrids parallels that of crossing the original parent plants to produce experimental original-parent-derived hybrids. The only difference, of course, is that many clones are now available corresponding to the respective original parent plants.

For optimum practice, pollen from clones from the original parent plant that had been used to provide pollen for the crossing of individual original parent plants is utilized in the crossing of cloned parental lines. Correspondingly, clones of the plant that had been the female member of the pair are used as females in the crossing of the clones. The same technique of manual emasculation of one plant and fertilization (either manually or with insects) with pollen of the other as was used for the original crossings is followed.

The preferred technique is the obtaining of pollen from clones of the plant that contributed pollen for the original crossing, and the corresponding use of emasculated clones of the plant that was the original pollen receptor. While this may, in theory, be unnecessary due to the reciprocity rule of plant genetics, it is the preferable approach in order to minimize any chance variations. In practice, the more vigorous parent will usually be employed as the female to maximize seed yield.

### 4.129  Verification By Field Trials

Before full scale commercial production of hybrid seeds can begin, it is highly desirable that semi-commercial field trials be conducted to verify the genetic equivalence between cloned-parent-derived hybrids and original-parent-derived hybrids. Thus, should it happen that for some inexplicable reason the hybrids obtained by crossing the clones are not of a quality corresponding to those obtained by crossing the individual original parent plants, commercial development of the hybrid can be abated.

Field trials to verify the equivalency are conducted using hybrid seeds obtained by crossing clones of the respective original parent plants, of a sufficient scale to provide the quantity of seeds necessary for field trials using commercial harvesting equipment. Any problems that occur can then be fetected before full scale commercial production begins.

### 4.1291  Commercial Production

Once cloned-parent-derived hybrids have been grown to maturity and harvested to provide for pre-commercial verification, full scale commercial production of hybrid seeds can begin.

The procedures of cloning the individual original parent plants by vegetative propagation that have been described earlier are repeated to obtain additional cloned parent plants. The clones of the respective original parents are then crossed to produce large quantities of hybrid seed for sale.

In the examples given heretofore, commercial hybrid seed production was accomplished by crossing two cloned-parent lines. For the purposes of this invention it is only necessary to clone those parent lines that are not homozygous (heterozygous). If one line should happen to be homozygous, it can be grown from seed or cloned. All heterozygous parent lines must, however, be clones of the original experimental parent plant.

4.13    Example 2.    Cabbage

4.131    Schedule

A typical schedule for the commercial production in California of high purity hybrid cabbage seed is presented below. Individual operations conducted according to the schedule are also discussed.

(1)    Summer, First Year

In the summer of the first year, a row of about 50 feet of each inbred parental line (genotype) used in making the selected hybrid is sown. These will be thinned. Conventional farming practices are followed and the plants in each parental line are permitted to grow to full market maturity.

(2)  <u>Fall, first year</u>

In the fall of the first year, when the plants have reached market maturity, about ten plants from each parental line are selected for their morphological characteristics. The individual plants are labeled, as by physically tagging the plant.  Each of the selected plants is then prepared for transplanting by trimming to a stump. When the plants have regrown, they are lifted and individually potted, and then moved to the greenhouse.

When the individual plants are selected, explants of each of the ten plants are taken for vegetative cloning. The cloning procedure, detailed below, is conducted organogenetically, under suitable tissue growth conditions in an aseptic nutrient medium.  Alternatively, some of the explants may be stored, as for example under cryogenic, <u>e.g.</u>, Seibert U.S. 4,052,817.

(3)  <u>Spring, second year</u>

Each of the selected plants blooms from March through April.  Standard horticultural test procedures for determining maximum self-incompatibility are applied to identify the individuals in each line that have the strongest self-incompatibility.  The test, in substance, involves growing each of the plants to maturity without external pollination, and then determining which plants produce the smallest yield of seeds, indicating the highest resistance to self-pollination (self-incompatibility).  The results of these tests are available by mid-July of the second year.

(4)  <u>Summer, second year</u>

Based on the tests used to determine which individual plants of each parental line have the strongest self-incompatibility, an individual plant is selected within each parental line.

Explants previously taken from each of the two selected individuals that exhibited maximum self-incompatibility are vegetatively propagated, as set forth below, to provide about 50 clones of each plant. Propagation of explants from the nonselected plants are discontinued as soon as the selection has been made.

(5) Fall, second year

The regenerated clones are transplanted in the fall of the second year, and are crossed to make cloned-parent-derived hybrids on a pilot production scale.

The clones are planted so that the cloned parental lines are in close proximity to each other but are isolated from chance fertilization by pollen from extraneous plants. Crossing of the lines occurs by natural pollination. This is best effected in large cages screened to exclude extraneous insects, but permitting flies, bees, or other insects contained within the cages to cross-pollinate the parental lines.

(6) Fall, third year

By the fall of the third year, hybrid seed from the crossing of cloned parents is harvested. Some seed from the cloned-parent-derived hybrids is then sent to an appropriate climate, e.g., Florida, for planting. The resulting cloned-parent-derived hybrid cabbage may then be compared with original-parent-derived hybrid cabbage made by crossing (by bud pollination) the two selected individual parent plants.

(7)  Spring, fourth year

Some cloned-parent-derived hybrid seed from the pilot production, along with the original-parent-derived hybrid see, is sown in California to verify that the cloned-parent-derived hybrids are equivalent to the original-parent-derived hybrids.

(8)  Summer, fourth year

Upon verification that the cloned-parent-derived hybrids are genetically comparable to the original-parent-derived hybrids, production plans can be made for using the clones in large scale production of F1 hybrid seed.

Additional clones are parepared by tissue culture technique, this time on a large scale suitable for commercial production.  Procedures for cloning are set out below.

(9)  Fall, fourth year

In the fall of the fourth year, the commercial hybrid production fields are set out.  A plant density of about 8,000 plants per acre is used, and an equal number of plants from each of the parent lines is used. These are then naturally cross-pollinated by insects. Seed from these plants is harvested, and is ready for sale.  As indicated previously, since each of the cloned parent lines is sib-incompatible, and since the reciprocal crosses have been tested for genetic equivalency, the entire seed production is marketable.

## 4.132  Clonal Propagation of Cabbage

This Example reports the study of several hormonal and nutritional parameters influencing the _in vitro_ development of excised shoot apices of head cabbage, _Brassica oleracea_ L. Var. _capitata_.

An in vitro culture protocol which ensures a capability of yielding normal plants from isolated shoot apices of this important vegetable crop has not been available. In vitro shoot apex culture or meristem culture has previously been employed to obtain pathogen-free plants (G.M. Morrel, _Amer. Orchid Soci. Bull._ _29_, 495-497 (1960)), to asexually propagate plants (T. Murashige, M.N. Shabde, P.M. Hasegawa, F.H. Takatori, and J.B. Jones, _J. Amer. Soc. Hort. Sci._ _97_, 158-161 (1972)), and to study morphogenic response of apical meristematic tissues (E.Ball, _Growth_, _24_, 91-110 (1960), M. Shabde, and T. Murashige, "Hormonal requirements of excised _Dianthus caryophyllus_ L. shoot apical meristem in vitro," _Amer. J. Bot._, _64_, 443-448 (1977); H.S. Smith and T. Murashige, "In vitro development of the isolated shoot apical meristem of angiosperms," Amer. J. Bot., _57_, 562-568 (1970).)

Axillary buds were obtained from market mature field grown head cabbage, cultivar Golden Acre. Buds were disinfected by a 0.5-1.5 min. wash in 70-75% ethanol followed by soaking in 1% sodium hypochlorite for 7 min. and washing three times with sterile water. Shoot apices approximately 100-150 um in length were aseptically isolated from axillary buds using microdissection. Each apex, or apical meristem, consisted of the apical dome with 1-2 subadjacent leaf primordia.

Apices were cultured on sterile 15 ml agar medium contained in 60 x 15 mm petri dishes or in 25 ml agar medium in 25 x 150 mm tubes. The basal nutrient medium contained the standard MS salt formulation (T. Murashige and F. Skoog, "A revised medium for the rapid growth and bioassays with tobacco tissue culture," Physiol. Plant., 15, 473-497 (1962) plus the following constituents, in mg/l: myo-inositol, 100; thiamine.HCl, 0.4; sucrose, 30,000; and agar, 7,000. The following were provided in the basal medium as test addenda: IAA (indole acetic acid) as the auxin, kinetin (6-furfuryl-aminopurine) as the cytokinin, sodium phosphate ($NaH_2PO_4 \cdot H_2O$), and adenine sulfate.

Ten to fifteen apices were used per treatment. Culture conditions were 26 ± 2°C and light at 2,500 lux from (Sylvania) cool white lamps 16 hrs/day. Culture period for observations was up to 6 weeks. Plants derived from culture were transplanted to a peat/vermiculite mixture after removing agar by washing with tap water. To harden the plants a mist system was used for the first two weeks. Plants were grown in a greenhouse (80 + 4°C) to market maturity (full head development).

Auxin and Cytokinin Interactions
for Axillary Shoot Apex Development:

For various other plant species it has been shown that isolated apices require auxins and/or cytokinins for continued development into plants (H.S. Smith and T. Murashige, "In vitro development of the isolated shoot apical meristem of angiosperms," Amer. J. Bot. 57, 562-568 (1970)). Therefore, experiments were carried out to investigate hormonal requirements for the development of cabbage shoot apices.

The variables studied, and the preferred nutrient composition that has been defined, are shown in Table A, below.

TABLE A

Culture Medium Composition for Plant Development
from Isolated Shoot Apices of Cabbage

INGREDIENTS

Inorganic constitutents

Organic constitutents

| | |
|---|---|
| myo-inositol | 100 |
| thiamin.HCl | 4 |
| sucrose | 30,000 |
| agar | 7,000 |
| pH 5.7 | |

| Variables Studied: | Recommended Amount |
|---|---|
| kinetin (0 - 10-0 mg/l) | 3 |
| IAA      (0 - 10.0 mg/l) | 1 |
| adenine sulfate.$2H_2O$ (80 mg/l) | Omit |
| $NaH_2PO_4.H_2O$ (170 mg/l) | 170 |

Murashige, T., and F. Skoog, Physiol. Plant., 473-497, 15 (1962).

Initial studies determined the effect of IAA on shoot and leaf development from isolated cabbage apices with kinetin held constant at 3 mg/l (selected from preliminary experiments not described here). The results are shown in Table 2. Optimal concentration of IAA for leaf induction was in the broad range of 0.1-10 mg/l, whereas IAA did not appear to influence the number of shoots differentiated.

## TABLE B

### Effect of IAA on In Vitro Development of Shoot Apex of Cabbage

| IAA (mg/l) | Size of Shoots (mm) | No. of Leaves | No. of Shoots | No. of Roots |
|---|---|---|---|---|
| 0 | 8 ± 2.5 | 1.6 ± 0.4 | 1.4 ± 0.5 | 0 |
| 0.1 | 13 ± 1.8 | 3.3 ± 0.7 | 2.3 ± 0.8 | 0 |
| 0.3 | 26 ± 6.8 | 3.4 ± 0.6 | 1.7 ± 0.2 | 0 |
| 1.0 | 19 ± 3.9 | 3.4 ± 0.6 | 1.8 ± 0.3 | 0 |
| 3.0 | 24 ± 2.8 | 3.3 ± 0.6 | 1.7 ± 0.2 | 0 |
| 10.00 | 23 ± 2.8 | 3.2 ± 0.7 | 2.0 ± 0.5 | 1.5 ± 1.3 |

Kinetin: 3 mg/l

In the next set of experiments, influence of kinetin concentrations on isolated cabbage apices was tested, with 1 mg/l IAA held constant. As can be seen in Table C, optimal kinetin concentration for both shoot and leaf development from isolated apices was 3 mg/l. Lower concentrations of kinetin (below 0.3 mg/l) caused callus formation and suppressed normal shoot development. This observation agrees with Shabde and Murashige's finding (M. Shabde, and T. Murashige, "Hormonal requirements of excised Dianthus caryopyllus L. shoot opical meristem in vitro," Amer. J. Bot., 64, 443-448 (1977)), which suggests that the isolated meristem dome of Dianthus caryophylus L. (carnation) requires both IAA and kinetin for its development into a plant.

## TABLE C

#### Effect of Kinetin on In Vitro Development
#### of Axillary shoot Apex of Cabbage

| Kinetin | Size of Shoots (mm) | No. of Leaves | No. of Shoots | No. of Roots |
|---------|---------------------|---------------|---------------|--------------|
| 0       | 2.1 ± 0.7           | 1.4 ± 0.4     | 0.6 ± 0.2     | 0            |
| 0.1     | 2.4 ± 0.3           | 1.8 ± 0.2     | 0.9 ± 0.1     | 0            |
| 0.3     | 5.1 ± 1.1           | 1.4 ± 0.2     | 0.6 ± 0.2     | 0            |
| 1       | 6.7 ± 1.2           | 1.7 ± 0.5     | 1.0 ± 0.2     | 0            |
| 3       | 27.8 ± 7.6          | 3.3 ± 0.5     | 2.5 ± 0.3     | 0            |
| 10.     | 5.5 ± 0.5           | 2.0 ± 0.2     | 1.0 ± 0.1     | 0            |

IAA:  1 mg/l

#### Stimulation of Axillary Shoot Apex
#### Development by Sodium Phosphate:

Both adenine sulfate and sodium phosphate (monosodium form) have been employed in plant tissue culture medium as constituents to promote shoot organogenesis (T. Murashige, M.N. Shabde, P.M. Hasegawa, F.H. Takatori, and J.B. Jones, J. Amer. Soc. Hort. Sci. 97, 158-161, (1972)). Table D summarizes the effects of adenine sulfate and sodium phosphate in the presence of both IAA and kinetin on in vitro development of axillary shoot apices of cabbage.

TABLE D

Effect of IAA, Kinetin, Adenine Sulfate and $NaH_2PO_4$ on the
In Vitro Development of Axillary Shoot Apex of Cabbage[']

| Addenda* | Size of Shoots (mm) | No. of Leaves | No. of Shoots | No. of Roots |
|---|---|---|---|---|
| Kinetin + IAA | 15 ± 1.6 | 2.4 ± 0.7 | 2 ± 0.6 | 0 |
| Kinetin + IAA + $NaH_2PO_4$ | 24.5 ± 6.6 | 4.5 ± 1.4 | 2.8 ± 0.5 | 4.5 ± 1.0 |
| Kinetin + IAA + Adenine Sulfate | 10.4 ± 3.8 | 3 ± 1.2 | 1.6 ± 0.6 | 0 |
| Kinetin + IAA + $NaH_2PO_4$ + Adenine Sulfate | 17 ± 3.1 | 2 ± 0.7 | 2 ± 0.7 | 0 |

---

* IAA (1 mg/l, Kinetin (3 mg/l), Adenine Sulfate (80 mg/l), $NaH_2PO_4$ (212 mg/l) were provided in the basal medium.

Adenine sulfate at 80 mg/l repressed shoot initiation and inhibited the chlorophyllous coloration in leaves. Murashige et al. (T. Murashige, M.N. Shabde, P.M. Hasegawa, F.H. Takatori, and J.B. Jones, J. Amer. Soc. Hort. Sci. 97, 158-161 (1972) reported that 160 mg/l adenine sulfate was toxic to asparagus shoot apex development. Thus, adenine sulfate was excluded from the medium components for cabbage apex culture. In contrast, sodium phosphate at 170 mg/l promoted apex development and rooting. Rooting response in media containing sodium phosphate may be related to the enhanced vigor of the developing shoots.

## Establishing Mature Cabbage Plants Derived from In Vitro Axillary Shoot Apices

Using the medium presented in Table E, at least 70% of excised cabbage apices developed into plants. A separate rooting step was usually not necessary. However, the rate of root development was enhanced by transferring shoots at 4 weeks to the same medium minus kinetin. After two weeks on the minus kinetin medium the developing plants were transplanted to pots containing a peat/vermiculite mixture and kept in a greenhouse. Survival at the transplanting stage was more than 90% under the conditions described.

### TABLE E

### Culture Medium Composition for Plant Development from Isolated Axillary Shoot Apices of Cabbage[a]

| CONSTITUENTS | mg/l |
|---|---|
| **Inorganic Salts** | |
| MS Salts formulation | [b] |
| $NaH_2PO_4 \cdot H_2O$ | 170 |
| **Organic Substances** | |
| Myo-inositol | 100 |
| Thiamine.HCl | 0.4 |
| Kinetin | 3 |
| **Organic Substances** | |
| IAA | 1 |
| Sucrose | 30,000 |

TABLE E   (cont'd)


Complex Addenda

Agar (SIGMA grade)                    7,000


a/  pH of medium was adjusted to 5.7 with 0.1 N KOH
    and/or 0.1 N HCl before autoclaving.
b/  Murashige and Skoog (T. Murashige and F. Skoog,
    "A revised medium for the rapid growth and bioassays
    with tobacco tissue culture," Physiol. Plant.,
    473-497 (1962)).


The developmental sequence from a shoot apex to a greenhouse transplant has been studied.  After 1-2 weeks the shoot apex started to swell, and elongation of several chlorophyllous leaf primordia was ordered.  Further apex development continued up to 4-5 weeks.  Occasionally, formation of adventitious shoots from the developing apex was apparent.  After four weeks, several roots were generated from the basal portion of shoots.  After two months under greenhouse conditions, plants developed normal heads.  The plants derived using this method were morphologically uniform; the genetic variability of plants cloned by this method has not yet been examined at the chromosomal level.

The foregoing Example demonstrated that, in cabbage, the presence of both IAA and kinetin are necessary for development of organized shoots within 6 weeks.  At low concentrations of kinetin (below 0.3 mg/l) in medium containing IAA, callus formation was observed and normal shoot development was suppressed.  The optimum response range for IAA was quite broad (0.3-10.0 mg/l) whereas that for kinetin was narrow (3.0 mg/l).  Adenine sulfate does not appear to be a beneficial media component and it

appears to repress the rooting response. Sodium phosphate on the other hand promotes apex development and rooting. (Rooting response in media containing sodium phosphate may be related to the enhanced vigor of the developing shoots.) A separate rooting step was not necessary in the media shown in Table E: however, the rate of root induction was enhanced if shoots at 4 weeks were transferred to the same medium minus kinetin. After two weeks the developing plants were ready for transplanting to the greenhouse. Survival at the transplanting stage was quite high if moisture and humidity were high initially.

4.14    EXAMPLE 3.    CAULIFLOWER


4.141    Schedule


A typical schedule for the commercial production in California of high purity hybrid cauliflower seed is presented below.    Individual operations conducted according to the schedule are also discussed.


(1)    Summer, First Year


In the summer of the first year, a row of about 50 feet of each inbred parental line (genotype) used in making the selected hybrid is sown.    These will be thinned. Conventional farming practices are followed and the plants in each parental line are permitted to grow to full market maturity, which requires between about 60 and 120 days.


(2)    Fall, first year


In the fall of the first year, when the plants have reached market maturity, about ten plants from each parental line are selected for their morphological characteristics.    The individual plants are labeled, as by physically tagging the plant. Each of the selected plants is then prepared for transplanting by conventional cauliflower curd treatment.    When the plants have regrown, they are lifted and individually potted, and then moved to the greenhouse.


When the individual plants are selected explants of each of the ten plants are taken for vegetative cloning. The cloning procedure, detailed below, is conducted organogenetically, under suitable tissue growth conditions in an aseptic nutrient medium.    Alternatively, all or part of the explants may be stored, as for example under cryogenic conditions, e.g., Seibert U.S. 4,052,817.

(3) Spring, second year

Each of the selected plants blooms from March through April. Standard horticultural test procedures for determining maximum self-incapability are applied to identify the individuals in each line that have the strongest self-incompatibility. The test, in substance, involves growing each of the plants to maturity without external pollination, and then determining which plants produce the smallest yield of seeds, indicating the highest resistance to self-pollination (self-incompatibility). The results of these tests are available by mid-July of the second year.

(4) Summer, second year

Based on the tests used to determine which individual plants of each parental line have the strongest self-incompatibility, an individual plant is selected within each parental line.

Explants previously taken from each of the two selected individuals that exhibited maximum self-incompatibility are vegetatively propagated, as set forth below, to provide about 50 clones of each plant. Propagation of explants from the non-selected plants are discontinued as soon as the selection has been made.

(5) Fall, second year

The regenerated clones are transplanted in the fall of the second year, and are crossed to make cloned-parent-derived hybrids on a pilot production scale.

The clones are planted so that the cloned parental lines are in close proximity to each other but are isolated from chance fertilization by pollen from extraneous plants. Crossing of the lines occurs by natural pollination. This is best effected in large cages screened to exclude extraneous insects, but permitting flies, bees, or other insects contained within the cages to cross-pollinate the parental lines.

(6) Fall, third year

By the fall of the third year, hybrid seed from the crossing of cloned parents is harvested. Some seed from the cloned-parent-derived hybrids is then sent to an appropriate climage, e.g., Florida, for planting. The resulting cloned-parent-derived hybrid cauliflower may then be compared with original-parent-derived hybrid cauliflower made by crossing (by bud pollination) the two selected individual parent plants.

(7) Spring, fourth year

Some cloned-parent-derived hybrid seed from the pilot production, along with the original-parent-derived hybrid seed, is sown in California to verify that the cloned-parent-derived hybrids are equivalent to the original-parent-derived hybrids.

(8) Summer, fourth year

Upon verification that the cloned-parent-derived hybrids are genetically comparable to the original-parent-derived hybrids, production plans can be made for using the clones in large scale production of $F_1$ hybrid seed.

Additional clones are prepared by tissue culture technique, this time on a large scale suitable for commercial production. Procedures for cloning are set out below.

(9) <u>Fall, fourth year</u>

In the fall of the fourth year, the commercial hybrid production fields are set out. A plant density of about 8,000 plants per acre is used, and an equal number of plants from each of the parent lines is used. These are then naturally cross-pollinated by insects. Seed from these plants is harvested, and is ready for sale. As indicated previously, since each of the cloned parent lines is sib-incompatible, and since the reciprocal crosses have been tested for genetic equivalency, the entire seet production is marketable.

4.142 <u>CLONAL PROPAGATION OF CAULIFLOWER</u>

This Example illustrates <u>in vitro</u> propagation of cauliflower, <u>Brassica oleracea</u>, var. <u>Botrytis</u>.

<u>General Procedure</u>

Using shoots derived from meristematic tissues of cauliflower curds, experiments were carried out to determine the nutritional requirements of shoot development and multiplication from explants for organogenetic clonal propagation.

Tissue culture propagation of Brassica crops is particularly important as an alternative means of maintaining self or sib incompatible inbred parents for $F_1$ hybrid seed production (Anderson & Carstens, 1977). There have been reported instances of in vitro organogenesis and embryogenesis from callus tissue culture of cauliflower (Baroncelli et al., 1973; Pareck & Chandra). However, adventitious shoots originated from meristematic primordia of curds have been most extensively studied for the purpose of clonal propagation (Powe, 1969; Walkey et al., 1970; Crisp & Walkey, 1974; Gout, 1975).

Although several reports on in vitro clonal propagation of this crop are available, knowledge of suitable culture medium for multiplication of in vitro formed shoots is limited. Therefore, explant clones derived from cauliflower curds were established to determine their hormonal and nutritional requirements in vitro.

Based in part on Murashige's three-step propagation methods (Murashige, 1974), a protocol and medium for cauliflower propagation were developed and are summarized in Table I, below. The procedure was:

Stage 1. Establishment of axenic culture:
As noted earlier, curd tissues were employed as initial explants, from which shoots develop and are isolated. The culture medium for this stage utilized a simple basal nutrient gel medium, high in auxin and low in cytokinin), lacking $NaH_2PO_4$, to produce the vegetative shoots for Stage 2. (See Table I, below)

Stage 2. Maximize the rate of vegetative shoot multiplication: Shoots from Stage 1 were isolated and then multiplied in Stage 2.  The Stage 2 medium consisted of basal medium, $NaH_2PO_4$, 6 mg/l Kinetin, and 1.0 mg/l IAA. The multiplication rate using this medium was about 13 shoots/2 weeks when cultured in 25 x 150 mm tubes containing 25 M agar medium.  This stage was repeated many times in order to provide the shoots for Stage 3.

### Stage 3.  Rooting of vegetative shoots:

This stage utilized a medium containing 0.1 mg/l Kinetin and 1 mg/l IAA to promote root formation.  $NaH_2PO_4$ was excluded from the medium.  For the purpose of recovering normal photosynthetic activity in green explants, sucrose concentration was lowered to one half of the basal medium.

The cultures in each stage were kept under light intensity of 2,500 lux provided by (Sylvania) cool white lamps for 16 hrs/day at constant 26 + 2°C temperature.

### Preparation of Tissue Explants

Curds were obtained from mature, field-grown cauliflower plants.  Tissues were disinfected by a 30 second wash in 70% ethanol followed by soaking in 1% sodium hypochlorite for 7 minutes followed by three rinses in sterile water.  Curds were further dissected to 2-3 mm size.

0044723

## TABLE I

Medium Composition[a] for Clonal Propagation of Cauliflower
Explants at Three Stages of In Vitro Culture[b]

| Constituents (mg/l) | Stage 1 Establishment | Stage 2 Multiplication | Stage 3 Rooting |
|---|---|---|---|
| **Inorganic Salts** | | | |
| MS Salts formulation | c) | c) | c) |
| $NaH_2PO_4 \cdot H_2O$ | – | 170 | – |
| **Organic Substances** | | | |
| myo-inositol | 100 | 100 | 100 |
| Thiamine.HCl | 0.4 | 0.4 | 0.4 |
| Kinetin | 1 | 6 | 0.1 |
| IAA | 3 | 1 | 1 |
| Sucrose | 30,000 | 30,000 | 15,000 |
| **Complex Addenda** | | | |
| Agar | 7,000 | 7,000 | 7,000 |

a)  pH of medium was adjusted to 5.7 with 0.1 N KOH and/or
    o.1 N HCl before autoclaving.

b)  Initial tissue for each culture stage are curds for
    Stage 1, vegetative shoots for Stage 2, and adventi-
    tiously formed shoots for Stage 3.  See detailed
    description of each Stage in the text.

c)  T. Murashige & F. Skoog, "A revised medium for the
    rapid growth and bioassays with tobacco tissue culture,"
    Physiol. Plant, 15, 473-497 (1962).

Stage 1 -- Establishment of Axenic Culture

The dissected curds were inoculated to a basal agar medium (Stage 1) which contained Murashige & Skoog salts and the following constituents, in mg/l: IAA (indole acetic acid) 3; Kinetin (6-furfuryl-aminopurine) 1; myo-inositol, 100; thiamine HCl, 0.4; sucrose, 30,000; and (Sigma) agar, 7,000.

At least ten shoot explants were used per treatment. The hormonal and nutritional parameters tested in this investigation were IAA as an auxin, kinetin as a cytokinin, IBA (indole-3-butyric acid) as an auxin, NAA (naphthalene acetic acid) as an auxin, 2iP ($N^6-A^2$-isopentenyl adenine) as a cytokinin, adenine sulfate, and sodium phosphate (monosodium form). Cultures were kept under 16 hrs light at 2,500 lux from (Sylvania) cool white lamps at a constant 26 ± 2°C. Observation was made after two weeks of culture.

The pH was adjusted to 5.7 ± 0.1 using 0.1 N HCl prior to autoclaving. Tissue explants were cultured on 15 ml agar medium contained in 60 x 15 mm plastic petri dishes. After two to three weeks, vegetative shoots developed from the curd primordia.

After two weeks' culture of curd tissues on the agar medium, meristematic tissues developed into chlorophyllous shoots. Indefinite adventitious shoot multiplication may be achieved by simply propagating individual shoots on basal medium. These shoot explants serve as a stock culture for further experimentation.

Experiments were carried out using a basal medium containing 1 mg/l Kinetin and IAA as a variant.

Table II shows the influence of IAA concentration on shoot or root organogenesis from cauliflower shoot explants. The optimum IAA concentration for shoot development was found to be in the neighborhood of 1 mg/l, whereas higher IAA concentrations were required to stimulate root initiation.

## TABLE II

Influence of IAA Concentration on Shoot or
Root Induction from Cauliflower Shoot Explants.
Medium Contained 80 mg/l Adenine Sulfate and
170 mg/l $NaH_2PO_4$. Kinetin at 3 mg/l
(Variation: + S.E.)

| IAA (mg/l) | No. of Shoots | No. of Roots |
|---|---|---|
| 0 | 1.7 | 0 |
| 0.1 | 3.1 | 0 |
| 0.3 | 3.2 | 0 |
| 1.0 | 5.3 | 0.6 |
| 3.0 | 3.3 | 0.5 |
| 6.0 | 2.4 | 6.0 |
| 10.0 | 3.0 | 10.0 |

In the next set of experiments, IAA was kept constant at 1 mg/l and Kinetin concentrations were varied from 0-10 mg/l. In Table III, one can see the dramatic effect of Kinetin on both shoot and root organogenesis. Kinetin at higher than 1 mg/l promotes shoot formation, but inhibits root differentiation.

TABLE III

Influence of Kinetin Concentration on Shoot
or Root Formation of a Cauliflower Shoot Explant.
Medium Contained 80 mg/l Adenine Sulfate and
170 mg/l $NaH_2PO_4$.   IAA at a Constant 1 mg/l
($\overline{\text{Variation}}$: + S.E.)

| Kinetin (mg/l) | No. of Shoots | No. of Roots |
|----------------|---------------|--------------|
| 0              | 2.4           | 4.7          |
| 0.1            | 2.2           | 5.3          |
| 0.3            | 3.7           | 2.8          |
| 1.0            | 3.1           | 0.2          |
| 3.0            | 3.8           | 0.6          |
| 6.0            | 5.2           | 0            |
| 10.0           | 4.0           | 0            |

Other experiments using other growth regulators such as BA, 2iP or NAA were conducted (data are not presented). BA, when combined with IAA, promoted shoot differentiation as effectively as did Kinetin. 2iP was less effective than Kinetin.

A dramatic result was observed when NAA was supplied with either Kinetin, BA or 2iP. Large quantities of root hairs were found on the NAA medium, a response not observed in the IAA control.

Table IV summarizes the effect of adenine sulfate (80 mg/l) and NaHPO4 (170 mg/l) in the present of both IAA and Kinetin. For comparison purposes, the effect of IAA or Kinetin alone in the basal medium is also presented. Adenine sulfate appears to inhibit adventitious shoot initiation in cauliflower shoot explants. In contrast, $NaH_2PO_4$ in the absence of adenine sulfate stimulates shoot organogenesis.

Shoots obtained from the axenic culture of Stage 1 were separated from the tissue under sterile conditions, and were then used for Stage 2 multiplication.

## Stage 2 -- Shoot Multiplication

For rapid multiplication of vegetative shoots, the basal medium was supplemented with 170 mg/l NaH2PO4, 1 mg/l IAA and 6 mg/l Kinetin. See Table I, above. The rate of multiplication was about 13 shoots/2 weeks when a 25 ml agar medium in a 25 x 150 mm culture tube was used.

## Stage 3 -- Rooting of Vegetative Shoots

The rooting of propagated shoots was enhanced by employing the basal medium with a modification of IAA and Kinetin at 1 mg/l and 0.1 mg/l respectively. See Table I, above. The low Kinetin concentration promoted root development. For the purpose of recoving normal photosynthetic activity in green explants, sucrose concentration was covered to use half of the basal medium.

TABLE IV

Effect of IAA, Kinetin, Adenine Sulfate and $NaH_2PO_4$,
on In Vitro Initiation of Shoot or Root of A
Cauliflower Shoot Explant. IAA (1 mg/l),
Kinetin (3 mg/l), Adenine Sulfate (80 mg/l), and
$NaH_2PO_4$ (170 mg/l) Added to the Basal Medium
(Variation: $\pm$ S.E.)

| ADDENDA | No. of Shoots | No. of Roots |
|---|---|---|
| IAA | 1.7 | 14.6 |
| Kinetin | 4.6 | 0 |
| IAA + Kinetin | 3.0 | 0 |
| IAA + Kinetin + Adenine Sulfate | 3.6 | 0 |
| IAA + Kinetin + $NaH_2PO_4$ | 6.5 | 0 |
| IAA + Kinetin + Adenine Sulfate + $NAH_2PO_4$ | 4.5 | 0 |

## Planting of Plantlets

Cauliflower plantlets derived from culture
were transplanted to pots containing a peat/vermiculite
mixture in a greenhouse.

The plants were hardened off under a mist
providing high humidity during the first two weeks after
transplantation. This procedure evidently enhances epicu-
ticular wax development, sufficient stem and root development, and full photosynthetic capability as reported
elsewhere (Grout, 1975; Grout and Aston, 1977; Grout &
Aston, 1978). Survival of plants was more than 95%.

0044723

The clonal plants developed normal curds after about two months, and flowered at approximately the same time as the plants derived from seeds.

Thus, the present protocol for cauliflower plant propagation provides a potential for large scale propagation of inbred lines for seed production.

WE CLAIM:

1. A process for rapidly developing hybrids and commercially producing hybrid seeds, comprising:

(a) selecting a first parent plant and a second parent plant;

(b) crossing said first parent plant with said second parent plant to obtain original-parent-derived hybrids that are phenotypically uniform;

(c) cloning said first parent plant to produce a first cloned parental line; and

(d) crossing plants of said first cloned parental line with said second parent plant or with a second parental line produced therefrom to obtain hybrid seeds which yield hybrids that are phenotypically uniform, provided that when said second parent plant. is heterozygous and a second parental line produced therefrom is used in the crossing of step (d), said second parental line must be produced by cloning.

2. The process according to claim 1 wherein said second parent plant is heterozygous.

3. The process according to claim 1 wherein both of said first and second parent plants are heterozygous.

4. The process according to claim 1 wherein the crossing of step (d) is between said second parent plant and said first parental line.

5. The process according to claim 1 where in the crossing of step (d) is between said second parental line and said first parental line.

6. The process according to claim 1 wherein said second parent plant is homozygous and the second parental line produced therefrom is obtained by cloning, or inbreeding to produce said second parental line.

7. The process of claim 1 wherein cloning is achieved by vegetative propagation.

8. The process according to claim 1 wherein the pollen donor of the crossing of step (d) is derived from the pollen donor of the crossing of step (b).

9. A process for rapidly developing hybrids and commercially producing hybrid seeds in high yield, resulting in improved properties of hybrids therefrom, comprising:

(a) crossing pairs of parent plants to produce sets of experimental original-parent-derived hybrids; from

(b) selecting at least one set of optimum original-parent-derived hybrids from among said sets of experimental original-parent-derived hybrids;

(c) obtaining parental lines from each parent plant which produced the selected set of optimum original-parent-derived-hybrids by cloning any heterozygous parent plant and cloning or inbreeding any homozygous parent plant, and

(d) crossing the plants in said parental lines to produce hybrid seeds.

10. The process according to claim 9 wherein at least one of said parent plants is heterozygous.

11. The process according to claim 9 wherein both of said parent plants are heterozygous.

12. The process according to claim 9 wherein cloning is achieved by vegetative propagation.

13. The process according to claim 9 wherein the pollen donor of the crossing of step (d) is derived from the pollen donor of the crossing of step (a).

14. The process according to claims 1 or 9 wherein said plants are tomato plants.

15. A process for the commercial production of high purity hybrid Brassica seed, comprising:

(a) selecting a maximally self-incompatible parent plant;

(b) cloning said selected self-incompatible parent plant to produce a first cloned parental line that is sib-incompatible;

(c) crossing plants of said first cloned parental line with plants of a second parental line; and

(d) collecting high purity hybrid seed from said sib-incompatible plants

16. The process according to claim 15 wherein said second parental line is a cloned line of a second maximally self-incompatible parent plant, the plants in said second cloned parental line being sib-incompatible.

17. The process according to claim 15 wherein said parent plants are cabbage plants.

18. The process according to claim 15 wherein cloning is by vegetative propagation.

19. The process according to claim 17 wherein said cloning is effected by axenic culture of excised shoot from said parent plant by establishing said shoots in a nutrient medium containing an auxin and a cytokinin.

20. The process according to claim 15 wherein said parent plants are cauliflower plants.

21. The process according to claim 20 wherein said cloning is effected by axenic culture of excised meristematic tissue from said parent plants by establishing shoots from said tissue in a nutrient medium having high auxin and low cytokinin contents, multiplying said shoots in a phosphate supplemented nutrient medium having low auxin and high cytokinin contents, and rooting said multiplied shoots in a nutrient medium having low auxin and very low cytokinin contents.